# EUROPEAN PATENT SPECIFICATION

(11) **EP 2 177 508 B1**
(45) Date of publication and mention of the grant of the patent: **12.09.2012**
(21) Application number: 09172659.6
(22) Date of filing: 09.10.2009
(51) Int. Cl.: C07C 319/20, C07C 319/28, C07C 323/52, C22C 38/44

(54) **Process for producing 2-hydroxy-4-methylthiobutanoic acid**
Verfahren zur Herstellung von 2-Hydroxy-4-Methylthiobutansäure
Procédé de production de l'acide 2-hydroxy-4-méthylthiobutanoïque

(30) Priority: 10.10.2008 JP 2008263734
(43) Date of publication of application: 21.04.2010
(73) Proprietor: Sumitomo Chemical Company, Limited, Tokyo 104-8260 (JP)
(72) Inventor: Fujita, Kazuo, Ehime (JP); Onishi, Kozo, Ehime (JP); Koizumi, Yoshiyuki, Ehime (JP)
(74) Representative: Winter, Brandl, Fürniss, Hübner, Röss, Kaiser, Polte - Partnerschaft

(56) References cited:
- EP-A1- 1 266 885
- EP-A1- 1 921 043
- JP-A- 11 217 370
- JP-A- 2003 104 960
- US-A1- 2006 047 169
- DATABASE WPI Week 200781 Thomson Scientific, London, GB; AN 2007-877395 XP002604213 & JP 2007 238555 A (SUMITOMO CHEM CO LTD) 20 September 2007 (2007-09-20)

## Description

### BACKGROUND OF THE INVENTION

### Field of the Invention

The present invention relates to a process for producing 2-hydroxy-4-methylthiobutanoic acid from 2-hydroxy-4-methylthiobutanenitrile via 2-hydroxy-4-methylthiobutanamide. In particular, the invention relates to a material for an apparatus for use in a solid-liquid separation step which comprises neutralizing a reaction solution which contains 2-hydroxy-4-methylthiobutanoic acid, and separating the same to obtain an organic phase; concentrating the organic phase; and separating from the organic phase a residue containing an inorganic salt, which is left to remain in the organic phase.

### Description of the Related Art

2-Hyroxy-4-methylthiobutanoic acid useful as an additive to a feed is known to be obtained by a method which comprises the steps of hydrating 2-hydroxy-4-methylthiobutanenitrile in the presence of sulfuric acid to obtain 2-hydroxy-4-methylthiobutanamide, and hydrolyzing 2-hydroxy-4-methylthiobutanamide to obtain 2-hydroxy-4-methylthiobutanoic acid (cf. JP-A-2007-238555). A similar process is disclosed in EP 1 266 885.

A nickel-based alloy such as Hastelloy^{®}C-22 is generally used, because of its corrosion resistance, as a material for an apparatus for use in a solid-liquid separation step wherein a reaction solution containing 2-hydroxy-4-methylthiobutanoic acid, obtained by hydration and hydrolysis reactions, is neutralized and phase-separated to obtain an organic phase, which is then concentrated, and a residue containing an inorganic salt, left to remain in the organic phase, is separated. Such alloy contains costly but essential elements of Mo and Ni in higher amounts.

### SUMMARY OF THE INVENTION

An object of the present invention is to provide a method of using corrosion resistant material containing lesser amounts of such elements for an apparatus for use in a solid-liquid separation step wherein a reaction solution containing 2-hydroxy-4-methylthiobutanoic acid, obtained by hydration and hydrolysis of 2-hydroxy-4-methylthiobutanenitrile, is neutralized and phase-separated to obtain an organic phase, which is then concentrated, and a residue containing an inorganic salt, left to remain in the organic phase, is separated. Thus, the present method is cost effective.

The present invention provides a process for producing 2-hydroxy-4-methylthiobutanoic acid, comprising carrying out
a solid-liquid separation step of separating a residue containing an inorganic salt from an organic phase concentrate containing 2-hydroxy-4-methylthiobutanoic acid, and the residue with an apparatus composed of, as a material,
an alloy which contains 21.0 to 30.0% by weight of a Cr element, 2.5 to 11.0% by weight of a Ni element, 1.0 to 5.0% by weight of a Mo element and a Fe element as the rest, at a temperature of from 60 to 95°C.

According to the present invention, there is provided a more inexpensive and corrosion resistant material for an apparatus for use in a solid-liquid separation step wherein a reaction solution containing 2-hydroxy-4-methylthiobutanoic acid, obtained by hydration and hydrolysis of 2-hydroxy-4-methylthiobutanenitrile, is neutralized and phase-separated to obtain an organic phase, which is then concentrated, and a residue containing an inorganic salt, left to remain in the organic phase, is separated; and thus, 2-hydroxy-4-methylthiobutanoic acid can be economically produced.

### Detailed Description of the Invention

### (Production Step)

2-Hydroxy-4-methylthiobutanoic acid (hereinafter optionally referred to as HMTBA) is produced by hydrating 2-hydroxy-4-methylthiobutanenitrile (hereinafter optionally referred to as HMTBN) in the presence of sulfuric acid usually at a temperature of from about 40 to about 70°C to obtain 2-hydroxy-4-methylthiobutanamide (hereinafter optionally referred to as HMTBAA), and adding water to 2-hydroxy-4-methylthiobutanamide to thereby hydrolyze the same usually at a temperature of from about 90 to about 130°C.
For example, HMTBN is industrially produced by reacting acrolein with methyl mercaptan to obtain 3-methylthiopropionaldehyde, and reacting this 3-methylthiopropionaldehyde with hydrogen cyanide.

Generally, HMTBAA is produced by feeding HMTBN, water and sulfuric acid to a hydration tank to hydrate HMTBN.
Fed to the hydration tank are usually about 20 to about 70 parts by weight, preferably about 25 to about 50 parts by weight of water per 100 parts by weight of HMTBN, and about 0.5 to about 1 mol in total, preferably about 0.6 to about 0.8 mol in total, of sulfuric acid per 1 mol of the HMTBN.

It is also possible to feed water in the form of a pre-mixture thereof with HMTBN and/or sulfuric acid, in other words, as an aqueous HMTBN solution and/or an aqueous sulfuric acid solution, to the hydration tank. Preferably, water is fed as an aqueous sulfuric acid solution to the hydration tank.
The hydration reaction is carried out usually at a temperature of about 40 to about 70°C for about 1 to about 3 hours. After the reaction, the reaction solution is typically aged. In the reaction solution, HMTBA is produced as a result of hydrolysis of a part of HMTBAA.

Then, the reaction solution containing HMTBAA as a main component, obtained in the hydration tank, is typically fed to a hydrolysis tank, and water is added to the reaction solution to thereby hydrolyze HMTBAA to produce HMTBA.
About 100 to about 200 parts by weight of water is usually fed per 100 parts by weight of the aqueous sulfuric acid solution in the above-described reaction solution.
In the hydrolysis tank, HMTBAA is typically hydrolyzed with water and sulfuric acid to produce HMTBA and concurrently to by-produce ammonium bisulfate (NH₄HSO₄) and ammonium sulfate ((NH₄)₂SO₄). After the addition of water and heating, the hydrolysis reaction is carried out at a temperature of from about 90 to about 130°C for about 2 to about 6 hours.
The hydration and hydrolysis reactions are typically carried out, using a glass-lined, resin-lined, MAT®21-made or AlloyB-2(Hastelloy® B-2)-made apparatus.

The reaction solution containing HMTBA, obtained in the hydrolysis tank, is usually distilled to remove low boiling point components from the reaction solution.
The distillation is carried out usually at a temperature of from about 80 to about 120°C under a pressure of from about 50 to about 150 kPa to remove by-produced low boiling point components such as dimethyl sulfide and formic acid. The low boiling point components are removed as a distillate usually at a rate of about 1 to about 4% by weight relative to the reaction solution, as required.
The removal of the low boiling point components may be done after neutralization or phase separation as will be described later.

### (Separation Step)

Then, an alkali is added to the reaction solution containing HMTBA, from which the low boiling point components have been removed, to thereby neutralize the reaction solution, and the reaction solution is phase-separated into an organic phase containing HMTBA and an aqueous phase containing water and an inorganic salt (containing ammonium bisulfate and ammonium sulfate). The neutralization and the phase separation are conducted, for example, using a mixer-settler type liquid-liquid extractor in which a stirrer tank and a phase-separation tank are combined as one set.

As the alkali, for example, sodium hydroxide, sodium hydrogencarbonate, sodium carbonate or the like is used in the form of an aqueous solution. The alkali is fed typically at a rate of from about 0.5 to about 1.2 mol, preferably from about 0.6 to about 0.8 mol, per 1 mol of ammonium bisulfate in the above-described reaction solution. The addition rate of the alkali may be controlled by a hydrogen ion concentration (pH) of the reaction solution admixed with the alkali.
The neutralization reaction is carried out usually at a temperature of from about 15 to about 120°C, preferably from about 30 to about 110°C, for about 0.1 to 3 hours, preferably about 0.1 to about 2 hours.

After the neutralization, the reaction solution is left to stand still in the phase-separation tank so as to phase-separate the reaction solution into an organic phase as an upper layer and an aqueous phase as a lower layer (liquid separation). The temperature for this separation is usually from about 30 to about 110°C.

### (Concentration Step)

The organic phase separated from the aqueous phase typically contains about 40 to about 60% by weight of HMTBA, about 20 to about 30% by weight of water and about 10 to about 30% by weight of an inorganic salt.
This organic phase is concentrated to remove the remaining water. For example, the concentration is done in a concentration tank usually at a temperature of from about 60 to about 150°C under a pressure of from about 1 to about 20 kPa, so that the water in the organic phase is reduced to about 5% by weight or less, preferably about 2% by weight or less, more preferably about 1% by weight or less. By this concentration, the sulfate ion concentration and kinematic viscosity of a product as will be described later can be decreased.
The inorganic salt is precipitated from the organic phase by this concentration, and the resulting organic phase forms a slurry.

When the residence time of the organic phase in the concentration tank is usually set to about 0.5 hour or longer, HMTBA is converted into an oligomer (which is mainly a dimer containing small amounts of a trimer and a tetramer). By doing so, the solubility of the inorganic salt in the organic phase is lowered, so that the concentration of the inorganic salt in the organic phase can be lowered. Preferably, the temperature, pressure and residence time are selected so that the weight ratio of the monomer of HMTBA to the oligomer of HMTBA can be about 2 to about 4. Such a concentration is effective to increase the particle size of the inorganic salt precipitated in the organic phase, so that a solid-liquid separation efficiency is improved during a solid-liquid separation as will be described later. Thus, the removal of the inorganic salt is facilitated.

### (Solid-Liquid Separation Step)

Then, the resultant slurry of the organic phase is cooled to usually about 60 to about 95°C, using a heat exchanger or the like. After that, the slurry is separated into a liquid component containing the organic phase and a solid component (or a residue) containing the precipitated inorganic salt.
The apparatus for use in the solid-liquid separation step refers to a solid-liquid separator, a pipe or a heat exchanger with which the slurry of the organic phase is brought into contact.

In the present invention, as a material for the apparatus for use in the solid-liquid separation step, employed is an alloy that contains 21.0 to 30.0% by weight of a Cr element, 2.5 to 11.0% by weight of a Ni element, 1.0 to 5.0% by weight of a Mo element and a Fe element as the rest.
SUS329J4L, SUS329J1, SUS329J3L, SCS10, and SCS11 exemplify commercially available alloys that contain 21.0 to 30.0% by weight of a Cr element, 2.5 to 11.0% by weight of a Ni element, 1.0 to 5.0% by weight of a Mo element and a Fe element as the rest.

A conventional nickel-based alloy contains about 13 to about 15% by weight of a Mo element and a Ni element as the rest (about 61 to about 65% by weight), while the alloy to be used in the present invention contains so small an amount as 1 to 5% by weight of a Mo element and a Fe element as the rest, and thus is inexpensive.

The solid-liquid separation is carried out typically at a temperature of from 60 to 95°C, preferably from 60 to 90°C. When this separation is carried out at a temperature higher than 95°C, the above-described alloy undesirably tends to corrode. When this separation is carried out at a temperature lower than 60°C, the viscosity of the slurry of the organic phase becomes higher, since the slurry obtained by the concentration in the concentration step has a relatively high viscosity and thus is low in filtration efficiency and deliquoring property. Thus, this is undesirable, since the solid-liquid separation becomes difficult.

As a solid-liquid separator, a centrifugal separator is usually used. The centrifugal separator may be of cylindrical type, separator plate type, decanter type or the like, among which a decanter type centrifugal separator is preferably used.

The separated liquid component comprises HMTBA (containing the oligomer produced during the concentration) as a main component. If needed, water is added to the liquid component to provide a product of HMTBA which typically contains about 88 to about 90% by weight of HMTBA, about 10 to about 12.5% by weight of a moisture content and very small amounts of other components.

### [Recovering Step]

The separated solid component contains about 20 to about 60% by weight of HMTBA, and thus is usually admixed with water to dissolve the inorganic salt, so as to phase-separate the solid component into an aqueous phase containing the inorganic salt and an organic phase. This organic phase is recovered. As the water to be added, the aqueous phase separated in the above-described separation step is preferably used. The organic phase to be recovered is mixed into the organic phase obtained during the above-described neutralization and phase-separation in the above-described separation step, so as to be recovered.

### Examples

An organic phase (HMTBA: 75% by weight, HMTBA dimer: 8.5% by weight, (NH₄)₂SO₄: 10% by weight, NH₄HSO₄: 0.5% by weight, and NaNH₄SO₄: 6% by weight) to be treated in the solid-liquid separation step was subjected to a corrosion test for the following metallic material.
The organic phase and a test piece of the following metallic material (a flat plate of 25 mm in length X 20 mm in width X 2 mm in thickness was U-like bent, and a stress was applied thereto (a U-bent test piece)) were put in a test container (a glass-made test bottle with a condenser used at 90°C or lower, or a glass-made autoclave used at 100°C or higher); the gas phase zone was purged with a nitrogen gas; and the test container was heated at a temperature indicated in Table 1 and was maintained at the same temperature for 168 hours. After that, the test piece was removed, rinsed and dried. Then, the reduced weight of the test piece was measured to determine a corrosion rate. The results are shown in Table 1.

### (Test Piece)

SUS329J4L (Cr: 24.9% by weight, Ni: 7.1% by weight, Mo: 3.1% by weight, N: 0.16 part by weight, and Fe: the rest)
AlloyC-22 (Hastelloy^{®} C-22) (Cr: 21.4% by weight, Mo: 13.3% by weight, and Ni: the rest)
AlloyC-276(Hastelloy^{®} C-276) (Cr: 15.1% by weight, Mo:
15.7% by weight, W: 3.6% by weight, and Ni: the rest)
Alloy59 (Cr: 22.9% by weight, Mo: 15.7% by weight, and Ni: the rest)

**[Table 1]**

| No. | Test piece | Temp. (°C) | Corrosion rate (mm/year) | Corroded state |
|---|---|---|---|---|
| 1 | SUS329J4L | 90 | 0.00 | Passivated surface |
| 2 | SUS329J4L | 100 | 0.10 | Active corroded surface |
| 3 | SUS329J4L | 120 | 0.06 | Active corroded surface |
| 4 | AlloyC-22 | 120 | 0.00 | Passivated surface |
| 5 | AlloyC-276 | 120 | 0.00 | Passivated surface |
| 6 | Alloy59 | 120 | 0.00 | Passivated surface |

AlloyC-22, AlloyC-276 and Alloy59 which were nickel-based alloys still had corrosion resistance even at 120°C.
Although SUS329J4L corroded at 100°C or higher, it did not corrode at 90°C.
Therefore, the use of an alloy which contains 21.0 to 30.0% by weight of a Cr element, 2.5 to 11.0% by weight of a Ni element, 1.0 to 5.0% by weight of a Mo element and a Fe element as the rest, as a material for the apparatus for use in the solid-liquid separation step at a temperature of from 60 to 95°C, is effective to realize economical production of 2-hydroxy-4-methylthiobutanoic acid without any corrosion of the apparatus.

## Claims

1. A process for producing 2-hydroxy-4-methylthiobutanoic acid, comprising carrying out
a solid-liquid separation step of separating a residue containing an inorganic salt, from an organic phase concentrate containing 2-hydroxy-4-methylthiobutanoic acid and the residue , with an apparatus composed of, as a material,
an alloy which contains 21.0 to 30.0% by weight of a Cr element, 2.5 to 11.0% by weight of a Ni element, 1.0 to 5.0% by weight of a Mo element and a Fe element as the rest, at a temperature of from 60 to 95°C.

2. A process according to claim 1, which comprises
a production step of producing 2-hydroxy-4-methylthiobutanoic acid by adding water and an acid to 2-hydroxy-4-methylthiobutanenitrile and/or 2-hydroxy-4-methylthiobutanamide;
a separation step of neutralizing a reaction solution which contains 2-hydroxy-4-methylthiobutanoic acid, obtained in the production step, by adding an alkali thereto, and phase-separating said reaction solution into an organic phase containing 2-hydroxy-4-methylthiobutanoic acid and an aqueous phase containing water and an inorganic salt;
a concentration step of concentrating 2-hydroxy-4-methylthiobutanoic acid by removing the remaining water from the organic phase separated in the separation step; and
the solid-liquid separation step.

3. The process of Claim 1 or 2, which comprises a recovering step of mixing the residue removed in the solid-liquid separation step, with the aqueous phase separated in the separation step; phase-separating the mixture into an organic phase containing 2-hydroxy-4-methylthiobutanoic acid and an aqueous phase containing water and an inorganic salt; and recovering the separated organic phase.

4. The process of Claim 1 or 2, wherein the alloy which contains 21.0 to 30.0% by weight of a Cr element, 2.5 to 11.0% by weight of a Ni element, 1.0 to 5.0% by weight of a Mo element and a Fe element as the rest is SUS329J4L, SUS329J1, SUS329J3L, SCS10 or SCS11.

## Patentansprüche

1. Verfahren zur Herstellung von 2-Hydroxy-4-methylthiobuttersäure, umfassend das Durchführen eines Fest-Flüssig-Trennungsschritts unter Abtrennen eines ein anorganisches Salz enthaltenden Rückstands von einer aufkonzentrierten organischen Phase, die 2-Hydroxy-4-methylthiobuttersäure und den Rückstand enthält, mit einer Vorrichtung, die als Material aus einer Legierung gebildet ist, die 21,0 bis 30,0 Gew.-% Cr-Element, 2,5 bis 11,0 Gew.-% Ni-Element, 1,0 bis 5,0 Gew.-% Mo-Element und als Rest Fe-Element enthält, bei einer Temperatur von 60 bis 95 °C.

2. Verfahren nach Anspruch 1, welches umfasst:
einen Herstellungsschritt zur Herstellung von 2-Hydroxy-4-methylthiobuttersäure durch Zugabe von Wasser und Säure zu 2-Hxdroxy-4-methylthiobuttersäurenitril und/oder 2-Hxdroxy-4-methylthiobuttersäureamid;
einen Trennungsschritt unter Neutralisieren einer Reaktionslösung, die bei dem Herstellungsschritt erhaltene 2-Hydroxy-4-methylthiobuttersäure enthält, durch Zugabe von Alkali, und Phasentrennung der Reaktionslösung in eine organische Phase, die 2-Hydroxy-4-methylthiobuttersäure enthält, und eine wässrige Phase, die Wasser und ein organisches Salz enthält;
einen Konzentrationsschritt unter Aufkonzentrieren von 2-Hydroxy-4-methylthiobuttersäure durch Entfernen des verbleibenden Wassers aus der bei dem Trennungsschritt abgetrennten organischen Phase; und
den Fest-Flüssig-Trennungsschritt.

3. Verfahren nach Anspruch 1 oder 2, umfassend einen Rückgewinnungsschritt unter Mischen des bei dem Fest-Flüssig-Trennungsschritt entfernten Rückstands mit der bei dem Trennungsschritt abgetrennten wässrigen Phase;
Phasentrennung der Mischung in eine organische Phase, die 2-Hydroxy-4-methylthiobuttersäure enthält, und eine wässrige Phase, die Wasser und ein organisches Salz enthält; und Rückgewinnen der abgetrennten organischen Phase.

4. Verfahren nach Anspruch 1 oder 2, wobei die Legierung, die 21,0 bis 30,0 Gew.-% Cr-Element, 2,5 bis 11,0 Gew.-% Ni-Element, 1,0 bis 5,0 Gew.-% Mo-Element und als Rest Fe-Element enthält, SUS329JAL, SUS329J1, SUS329J3L, SCS10 oder SCS11 ist.

## Revendications

1. Procédé de production d'acide 2-hydroxy-4-méthylthiobutanoïque, comprenant
la réalisation d'une étape de séparation solide-liquide pour séparer un résidu contenant un sel inorganique d'un concentré en phase organique contenant de l'acide 2-hydroxy-4-méthylthiobutanoïque et le résidu, avec un appareil composé, en tant que matériau,
d'un alliage qui contient de 21,0 à 30,0 % en poids d'un élément Cr, de 2,5 à 11,0 % en poids d'un élément Ni, de 1,0 à 5,0 % en poids d'un élément Mo et un élément Fe pour le reste, à une température allant de 60 à 95°C.

2. Procédé selon la revendication 1, qui comprend
une étape de production pour produire de l'acide 2-hydroxy-4-méthylthiobutanoïque en ajoutant de l'eau et un acide à du 2-hydroxy-4-méthylthiobutanenitrile et/ou du 2-hydroxy-4-méthylthiobutanamide ;
une étape de séparation pour neutraliser une solution de réaction qui contient l'acide 2-hydroxy-4-méthylthiobutanoïque, obtenu lors de l'étape de production, en y ajoutant un alcali, et séparer en phases ladite solution de réaction en une phase organique contenant de l'acide 2-hydroxy-4-méthylthiobutanoïque et en une solution aqueuse contenant de l'eau et un sel inorganique ;
une étape de concentration pour concentrer l'acide 2-hydroxy-4-méthylthiobutanoïque en éliminant l'eau restante de la phase organique séparée lors de l'étape de séparation ; et
l'étape de séparation solide-liquide.

3. Procédé selon la revendication 1 ou 2, qui comprend une étape de récupération du mélange du résidu éliminé lors de l'étape de séparation solide-liquide, avec la phase aqueuse séparée lors de l'étape de séparation ; la séparation en phases du mélange en une phase organique contenant de l'acide 2-hydroxy-4-méthylthiobutanoïque et en une phase aqueuse contenant de l'eau et un sel inorganique ; et la récupération de la phase organique séparée.

4. Procédé selon la revendication 1 ou 2, dans lequel l'alliage qui contient de 21,0 à 30,0 % en poids d'un élément Cr, de 2,5 à 11,0 % en poids d'un élément Ni, de 1,0 à 5,0 % en poids d'un élément Mo et un élément Fe pour le reste est SUS329J4L, SUS329J1, SUS329J3L, SCS10 ou SCS11.
